# EUROPÄISCHE PATENTSCHRIFT

(11) **EP 3 600 564 B1**
(45) Veröffentlichungstag und Bekanntmachung des Hinweises auf die Patenterteilung: **22.09.2021**
(21) Anmeldenummer: 18711045.7
(22) Anmeldetag: 06.03.2018
(51) Int. Cl.: A61Q 17/04, A61K 8/34, A61K 8/39

(54) **SONNENSCHUTZMITTEL MIT POLYGLYCERYL-10 STEARAT UND CITRONELLOL**
SUNSCREEN CONTAINING POLYGLYCERYL-10 STEARATE AND CITRONELLOL
PRODUIT DE PROTECTION SOLAIRE CONTENANT DU POLYGLYCÉRYL-10-STÉARATE ET DU CITRONELLOL

(30) Priorität: 22.03.2017 DE 102017204791
(43) Veröffentlichungstag der Anmeldung: 05.02.2020
(73) Patentinhaber: Beiersdorf AG, 20253 Hamburg (DE)
(72) Erfinder: SPROCK, Sarah, 22297 Hamburg (DE); GÖDDERTZ, Dominik, 22549 Hamburg (DE); SCHADE, Tatjana, 25866 Mildstedt/Rosendahl (DE)
(74) Vertreter: Beiersdorf AG
(86) Internationale Anmeldenummer: PCT/EP2018/055411
(87) Internationale Veröffentlichungsnummer: WO 2018/172056

(56) Entgegenhaltungen:
- EP-A1- 3 093 009
- DE-A1-102014 206 156
- DATABASE GNPD [Online] MINTEL; 1. Juli 2014 (2014-07-01), BB Hydra+ Beauty Balm SPF 25: XP002780244, Database accession no. 2314138
- DATABASE GNPD [Online] MINTEL; 1. Dezember 2012 (2012-12-01), "Perfecting Anti-Wrinkle Correction Cream", XP002780245, Database accession no. 1961962

## Beschreibung

Die vorliegende Erfindung betrifft eine kosmetische Zubereitung enthaltend ein oder mehrere UV-Filter, Polyglyceryl-10 Stearat (INCI Polyglyceryl-10 Stearate) und Citronellol, sowie ein Verfahren zur Erhöhung der Auswaschbarkeit von UV-Filtern aus Textilien, die mit einer kosmetischen Zubereitung, welche diese UV-A Filter und/oder Breitbandfilter enthält, kontaminiert sind, wobei der Zubereitung Polyglyceryl-10 Stearat (INCI Polyglyceryl-10 Stearate) und Citronellol zugesetzt wird und die Verwendung einer Mischung aus Polyglyceryl-10 Stearat (INCI Polyglyceryl-10 Stearate) und Citronellol in UV-Lichtschutzfilter enthaltenden kosmetischen Zubereitungen zur Erleichterung der Auswaschbarkeit der UV-Lichtschutzfilter aus mit den Zubereitungen kontaminierten Textilien.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter wie 4-(tert.-Butyl)-4'-methoxydibenzoylmethan (INCI Butyl Methoxydibenzoylmethane), (2-[-4-(Diethylamino)-2-hydroxybenzoyl] Benzoesäurehexylester (INCI: Diethylamino Hydroxybenzoyl Hexyl Benzoate) und 2,4-Bis-{[4-(2-ethyl-hexyloxy)-2-hydroxyl-phenyl}-6-(4-methoxyphenyl)-1,3,5-triazin (INCI: Bis-Ethylhexyloxyphenol Methoxyphenyl Triazin) hervorgerufen. Die Verfleckungen sind durch Waschen mit herkömmlichen Waschmitteln kaum zu entfernen und verstärken sich während des Waschprozesses durch Wechselwirkungen mit lonen des Waschwassers sogar noch.

Es war daher die Aufgabe der vorliegenden Erfindung, die Nachteile des Standes der Technik zu beseitigen und eine kosmetische Zubereitung (insbesondere ein Sonnenschutzmittel) zu entwickeln, die nicht-wasserlösliche UV-A-Filter wie 4-(tert.-Butyl)-4'-methoxydibenzoylmethan (INCI Butyl Methoxydibenzoylmethane) und (2-[-4-(Diethylamino)-2-hydroxybenzoyl] Benzoesäurehexylester (INCI: Diethylamino Hydroxybenzoyl Hexyl Benzoate) sowie gegebenenfalls Breitbandfilter wie Bis-Ethylhexyloxyphenol Methoxyphenyl Triazine enthält, welche sich leichter aus den mit der Zubereitung kontaminierten Textilen herauswaschen lassen.

Überraschend gelöst werden die Aufgaben durch eine kosmetische Zubereitung enthaltend
a) ein oder mehrere UV-Filter,
b) Polyglyceryl-10 Stearat (INCI Polyglyceryl-10 Stearate),
c) Citronellol, wobei die Zubereitung frei ist von 2-Hydroxy-4-methoxybenzophenon, 4-Methoxyzimtsäure(2-ethylhexyl)ester, 4-Methoxyzimtsäureisoamylester und 3-(4-Methylbenzyliden)campher,

Überraschend gelöst werden die Aufgaben ferner durch ein Verfahren zur Erhöhung der Auswaschbarkeit von organischen, öllöslichen UV-A Filtern und/oder organischen, öllöslichen Breitbandfiltern aus Textilien, die mit einer kosmetischen Zubereitung, welche diese UV-A Filter und/oder Breitbandfilter enthält, kontaminiert sind, dadurch gekennzeichnet, dass der kosmetischen Zubereitung Polyglyceryl-10 Stearat (INCI Polyglycoryl-10 Stearate) und Citronellol zugesetzt wird, wobei die Zubereitung frei ist von 2-Hydroxy-4-methoxybenzophenon, 4-Methoxyzimtsäure(2-ethylhexyl)ester, 4-Methoxyzimtsäureisoamylester und 3-(4-Methylbenzyliden)campher.

Überraschend gelöst werden die Aufgaben auch durch ein Verfahren zur Reduzierung der durch UV-Lichtschutzfilter enthaltenden kosmetischen Zubereitungen hervorgerufenen Textilverfleckung, dadurch gekennzeichnet, dass dem Kosmetikum Polyglyceryl-10 Stearat (INCI Polyglyceryl-10 Stearate) und Citronellol zugesetzt wird, wobei die Zubereitung frei ist von 2-Hydroxy-4-methoxybenzophenon, 4-Methoxyzimtsäure(2-ethylhexyl)ester, 4-Methoxyzimtsäureisoamylester und 3-(4-Mathylbenzyliden)campher,

Nicht zuletzt werden die Aufgaben gelöst durch die Verwendung von einer Mischung aus Polyglyceryl-10 Stearat (INCI Polyglyceryl-10 Stearate) und Citronellol in UV-Lichtschutzfilter enthaltenden kosmetischen Zubereitungen zur Erleichterung der Auswaschbarkeit der UV-Lichtschutzfilter aus mit den Zubereitungen kontaminierten Textilien, wobei die Zubereitung frei ist von 2-Hydroxy-4-methoxybenzophenon, 4-Methoxyzimtsäure(2-ethylhexyl)ester, 4-Methoxyzimtsäureisoamylester und 3-(4-Methylbenzyliden)campher.

Zwar kennt der Stand der Technik die DE 102014216602, DE 102014202956, DE 102013213170, DE 102013200819, DE 102011088962 und EP 2937073, Auch gibt es den nachveröffentlichten Stand der Technik der DE102017200723 doch konnten diese Schriften nicht den Weg zur vorliegenden Erfindung weisen. Darüber hinaus kennt der Fachmann die Datenbank-Einträge in der GNPD-Datenbank Mintel "BB Hydra+Beauty Balm SPF 25", Eintragungsnummer 2314138 und "Perfecting Anti-Wrinkle Correction Cream" Eintragungsnummer 1961962, sowie die die DE 102014206156 A1 und EP 3093009 A1, die ebenfalls nicht den Weg zur vorliegenden Erfindung weisen konnten.

Im Rahmen der vorliegenden Offenbarung beziehen sich die Formulierungen "erfindungsgemäß", "erfindungsgemäße Zubereitung" etc. immer auf die erfindungsgemäßen Zubereitungen, Verfahren und Verwendungen, d.h. auch auf Zubereitungen, in denen die erfindungsgemäßen Verwendungen verwirklicht werden sowie Zubereitungen, mit denen das erfindungsgemäße Verfahren verwirklicht wird.

Es ist erfindungsgemäß vorteilhaft, wenn die erfindungsgemäße Zubereitung in Form einer Emulsion vorliegt. Dabei ist es erfindungsgemäß bevorzugt, wenn die Zubereitung in Form einer ÖI-in-Wasser-Emulsion (O/W-Emulsion) vorliegt. Erfindungsgemäß besonders bevorzugt liegt die O/W-Emulsion in Form einer Lotion vor.

Erfindungsgemäß vorteilhafte Ausführungsformen der vorliegenden Erfindung sind dadurch gekennzeichnet, dass die Zubereitung Polyglyceryl-10 Stearat in einer Konzentration von 0.1 bis 1.5 Gewichts-%, bezogen auf das Gesamtgewicht der Zubereitung enthält, Dabei ist es erfindungsgemäß bevorzugt, wenn dass die Zubereitung Polyglyceryl-10 Stearat in einer Konzentration von 0.3 bis 1.0 Gewichts-%, bezogen auf das Gesamtgewicht der Zubereitung enthält.

Erfindungsgemäß besonders bevorzugte Ausführungsformen enthalten neben Polyglyceryl-10 Stearat zusätzlich Glyceryistearat. In einem solchen Fall ist es erfindungsgemäß von Vorteil Glycerylstearat in einer Konzentration von 0.1 bis 2.0 Gewichts-%, bezogen auf das Gesamtgewicht der Zubereitung, einzusetzen.

Es ist erfindungsgemäß vorteilhaft, wenn die erfindungsgemäße Zubereitung Citronellol in einer Konzentration von 10 ppm bis 0,3 Gewichts-%, bezogen auf das Gesamtgewicht der Zubereitung enthält. Die erfindungsgemäß bevorzugte Einsatzkonzentration für Citronellol liegt bei einer Konzentration von 40 ppm bis 0.2 Gewichts-%, bezogen auf das Gesamtgewicht der Zubereitung.

Vorteilhaft im Sinne der vorliegenden Erfindung sind Zubereitungen, die dadurch gekennzeichnet sind, dass die Zubereitung Phenoxyethanol enthält.
In einem solchen Fall ist es erfindungsgemäß von Vorteil Phenoxyethanol in einer Konzentration von 0.1 bis 1.0 Gewichts-%, bezogen auf das Gesamtgewicht der Zubereitung, einzusetzen.

Erfindungsgemäß vorteilhaft ist es, wenn die Zubereitung Cellulosegummi (INCI Cellulose gum) und/oder Xanthangummi (INCI Xanthan gum) enthält. Dabei ist ein Gehalt von Cellulosegummi und Xanthangummi erfindungsgemäß bevorzugt.

Es ist es erfindungsgemäß von Vorteil Cellulosegummi (INCI Cellulose gum) in einer Konzentration von 0.05 bis 1.0 Gewichts-%, bezogen auf das Gesamtgewicht der Zubereitung, einzusetzen.

Die erfindungsgemäß vorteilhafte Einsatzkonzentration für Xanthangummi beträgt von 0.1 bis 1.0 Gewichts-%, bezogen auf das Gesamtgewicht der Zubereitung.

Erfindungsgemäß vorteilhafte Ausführungsformen der vorliegenden Erfindung sind dadurch gekennzeichnet, dass die Zubereitung Tetranatriumiminodisuccinat (INCI Tetrasodium Iminodisuccinate) enthält.

Es ist erfindungsgemäß vorteilhaft, wenn die erfindungsgemäße Zubereitung Tetranatriumiminodisuccinat (INCI Tetrasodium Iminodisuccinate) in einer Konzentration von 0.01 bis 1.5 Gewichts-%, bezogen auf das Gesamtgewicht der Zubereitung enthält. Die erfindungsgemäß bevorzugte Einsatzkonzentration für Tetranatriumiminodisuccinat (INCI Tetrasodium Iminodisuccinate) liegt bei einer Konzentration von 0.01 bis 1.0 Gewichts-%, bezogen auf das Gesamtgewicht der Zubereitung

Erfindungsgemäß ganz besonders bevorzugt ist es, eine Kombination aus Cellulosegummi (INCI Cellulose gum) und Tetranatriumiminodisuccinat (INCI Tetrasodium Iminodisuccinate) einzusetzen, wobei die oben genannten Konzentrationsbereiche die erfindungsgemäß bevorzugten Einsatzkonzentrationen darstellen.

Vorteilhaft im Sinne der vorliegenden Erfindung sind Zubereitungen, die dadurch gekennzeichnet sind, dass die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

Dabei ist insbesondere der Einsatz von Ethylhexylglycerin erfindungsgemäß besonders vorteilhaft.
Die erfindungsgemäß vorteilhafte Einsatzkonzentration für Ethylhexylglycerin beträgt von 0.1 bis 1 Gewichts-%, bezogen auf das Gesamtgewicht der Zubereitung.

Erfindungsgemäß bevorzugt ist der Einsatz einer Kombination aus Phenoxyethanol und Ethylhexylglycerin.

Erfindungsgemäß besonders bevorzugt ist der Einsatz einer Kombination aus Phenoxyethanol, Ethylhexylglycerin und Ethanol.

Die erfindungsgemäß vorteilhafte Einsatzkonzentration für Ethanol beträgt von 0.5 bis 10.0 Gewichts-%, bezogen auf das Gesamtgewicht der Zubereitung.

Erfindungsgemäß bevorzugte Ausführungsformen werden dadurch erhalten, dass die Zubereitung keine Parabene, sowie kein Methylisothiazolinon, Chlormethylisothiazolinon und DMDM-Hydantoin enthält, also frei ist von diesen Inhaltsstoffen.

Es ist erfindungsgemäß vorteilhaft, wenn die Zubereitung einen oder mehrere UV-Filter gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan, 2-Phenylbenzimidazol-5-sulfonsäuresalzen, 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine), 4,4'-[[6-[[4-[[(1,1 dimethylethyl)amino]carbonyl]phenyl] amino]-1,3,5-triazine-2,4-diyl]diimino]bis-, bis(2-ethylhexyl)benzoat (INCI: Diethylhexyl Butamido Triazone), 2-Ethylhexyl 2-hydroxybenzoat (INCI: Ethylhexyl Salicylate), 3,3,5-Trimethylcyclohexyl 2-hydroxybenzoat (INCI: Homosalate), Titandioxid enthält.

Erfindungsgemäß bevorzugt ist es, wenn die Zubereitung einen oder mehrere UV-Filter gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert,-Butyl)-4'-methoxydibenzoylmethan, 2,4-Bis-{[4-(2-ethyl-hexyloxy)-2-hydroxy]-phenyl)-6-(4-methoxyphenyl)-1,3,5-triazin (INCI: Bis- Ethylhexyloxyphenol methoxyphenyl Triazine), enthält.

Enthält die erfindungsgemäße Zubereitung Hexyl 2-[4-(diethylamino)-2-hydroxybenzoyl]benzoate (INCI: Diethylamino hydroxybenzoyl hexyl benzoate), so ist es erfindungsgemäß vorteilhaft, diese Verbindung in einer Konzentration von 0,1 bis 10 Gewichts-%, bezogen auf das Gesamtgewicht der Zubereitung einzusetzen.

Enthält die erfindungsgemäße Zubereitung 4-(tert.-Butyl)-4'-Mathoxydibenzoylmethan, so ist es erfindungsgemäß vorteilhaft, diese Verbindung in einer Konzentration von 0.1 bis 5.0 Gewichts-%, bezogen auf das Gesamtgewicht der Zubereitung einzusetzen.

Enthält die erfindungsgemäße Zubereitung 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine), so ist es erfindungsgemäß vorteilhaft, diese Verbindung in einer Konzentration von 0.1 bis 10.0 Gewichts-%, bezogen auf das Gesamtgewicht der Zubereitung einzusetzen.

Erfindungsgemäß vorteilhaft enthält die erfindungsgemäße Zubereitung Natriumpolyacrylat (INCI Sodium Polyacrylate).

Erfindungsgemäß vorteilhafte Ausführungsformen der vorliegenden Erfindung sind dadurch gekennzeichnet, dass die Zubereitung Titandioxid enthält.

Erfindungsgemäß besonders bevorzugt ist ein Titandioxid in der Kristallstruktur Rutil mit einer Primärpartikelgröße von 2 -100 nm.
Es ist erfindungsgemäß ganz bevorzugt, wenn die Primärpartikelgröße des erfindungsgemäßen Titandioxides im Bereich zwischen 5 und 50 nm liegt.

Erfindungsgemäß vorteilhafte Ausführungsformen der vorliegenden Erfindung sind dabei dadurch gekennzeichnet, dass das Titandioxid mit Silica (Siliziumdioxid und/oder Kieselsäure) beschichtet ist.

Es ist dabei erfindungsgemäß bevorzugt, wenn das mit Silica beschichtete Titandioxid auf der äußeren Seite der Silica-Schicht (d.h. auf der dem Titandioxid-abgewandten Seite) eine Schicht aus Dimethicone, Simethicone oder Methicone aufweist. Unter diesen Stoffen ist Dimethicone dabei erfindungsgemäß besonders bevorzugt.

Es ist erfindungsgemäß vorteilhaft, wenn die sekundäre Partikelgröße des Titandioxid in der Kristallstruktur Rutil mit einer Primärpartikelgröße von 2 -100 nm zwischen 0,05 und 50 µm beträgt. Dabei ist der Bereich von 0,1 bis 1 µm erfindungsgemäß bevorzugt.

Die primäre und sekundäre Partikelgröße entnimmt der Fachmann folgender Literaturstelle: SCCS/1516/13 Opinion on Titanium Dioxide (nano form) Colipa No S75 der Cosmetics Europe personal care association.

Erfindungsgemäß vorteilhafte Ausführungsformen der vorliegenden Erfindung sind dadurch gekennzeichnet, dass die Zubereitung einen oder mehrere der Parfümstoffe Hexylsalicylat, Linaylacetat, 2-Isobutyl-4-hydroxy-4-methyltetrahydropyran, Adipinsäurediester, Methylheptenon, alpha-Isomethylionon, Butylphenylmethylpropioal, Coumarin, Hexylcinnamal, Limonen, Diethylsuccinat, Hydroxyisohexyl 3-Cyclohexencarboxaldehyd, Diethylsuccinat, Menthyl PCA und Citronellylmethylcrotonat, Benzyl Benzoate, Alpha-Isomethylionon, Benzylalkohol, Benzylcinnamat, Benzylsalicylat, Linalool, Eugenol, Geraniol enthält.

Die erfindungsgemäße Zubereitung stellt üblicherweise eine ÖI-in-Wasser-Emulsion (O/W-Emulsion) dar. Diese kann die für derartige Zubereitungen üblichen Inhaltsstoffe enthalten.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum und/oder Polyethylen, Nylon, Silica Diemthyl Silyate.

Es ist erfindungsgemäß bevorzugt, wenn die erfindungsgemäße Zubereitung Silica Dimethyl Silylate enthält.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist es erfindungsgemäß bevorzugt, wenn die Zubereitung Dibutyladipat, Dicaprylylcarbonat und/oder C12-C15 Alkylbenzoat enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Es ist dabei erfindungsgemäß bevorzugt, wenn die Zubereitung Xanthangummi, quervernetztes Acrylat/C10-C30 Alkylacrylat Polymer und/oder Vinylpyrrolidon/Hexadecen-Copolymer enthält.

Ein Gehalt an Glycerin von mindestens 0.5 Gewicht-%, bezogen auf das Gesamtgewicht der Zubereitung, ist erfindungsgemäß besonders vorteilhaft.

Darüber hinaus ist es erfindungsgemäß von besonderem Vorteil, wenn die erfindungsgemäße Zubereitung Ethanol enthält.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Glycyrrhitinsäure, Polidocanol, natürliche und/oder synthetische Isoflavonoide, Flavonoide, Kreatin, Kreatinin, Taurin, ß-Alanin, Panthenol, Magnolol, Honokiol, Tocopherol, Tocopherylacetat, Dihydroxyaceton; 8-Hexadecen-1,16-dicarbonsäure, Glycerylglycose, (2-Hydroxyethyl)harnstoff, Hyaluronsäure und/oder deren Salze und/oder Licochalcon A enthält.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im Wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 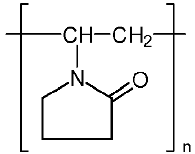 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79), Acrylates Copolymer (Epitex 66). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

### Vergleichsversuch

Mit Hilfe des folgenden Vergleichsversuches konnte der erfinderische Effekt beispielhaft belegt werden: Es wurden die folgenden Rezepturen hergestellt und die Auswaschbarkeit aus Textilien bestimmt.

Es wurden die folgenden Rezepturen hergestellt:

| **inci** | **Referenz** | **mit Citronellol** |
|---|---|---|
| Citronellol | 0 | 0,3 |
| Tocopheryl Acetate | 0,06 | 0,06 |
| Glycyrrhetinic Acid | 0,1 | 0,1 |
| Ethylhexylglycerin | 0,15 | 0,15 |
| Hydrogenated Coco-Glycerides | 1 | 1 |
| Polyglyceryl-10 Stearate | 0,8 | 0,8 |
| Glyceryl Stearate | 1 | 1 |
| Silica Dimethyl Silylate | 1 | 1 |
| Tapioca Starch + Aqua | 1 | 1 |
| VP/Hexadecene Copolymer | 0,5 | 0,5 |
| Glycerin + Aqua | 0,9 | 0,9 |
| Phenoxyethanol | 0,3 | 0,3 |
| Behenyl Alcohol | 0,5 | 0,5 |
| Cellulose Gum | 0,5 | 0,5 |
| Hydroxyethylcellulose | 0,2 | 0,2 |
| Xanthan Gum | 0,4 | 0,4 |
| Sodium Polyacrylate | 0,1 | 0,1 |
| Aqua | 57,23 | 56,93 |
| Alcohol Denat. + Aqua | 7 | 7 |
| Aqua + Trisodium EDTA | 1 | 1 |
| Tetrasodium Iminodisuccinate | 0,01 | 0,01 |
| Homosalate | 9,5 | 9,5 |
| Butyl Methoxydibenzoylmethane | 4,75 | 4,75 |
| Octocrylene | 8 | 8 |
| Titanium Dioxide (nano) + Silica + Dimethicone | 4 | 4 |

### Messung der Auswaschbarkeit

Es wurden die vier Sonnenschutzemulsionen hinsichtlich der Bildung von gelben Flecken auf Baumwolltextilien über einen in vitro Auftragungs-/ Waschzyklus untersucht. Es wurden dabei weiße vorgewaschene Baumwollmonitore (100% Baumwolle) verwendet.

Dazu wurden je 3 mg/cm² der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät DATACOLOR 800 (Datacolor International).
Messgeometrie: d/8°, SCE (Glanzkomponente ausgeschlossen)
Lichtart/Beobachter: D65/10°(entsprechend mittleres Tageslicht)
UV-Filterung: an D65 angepasst, Ganz/Griesser Methode
Messöffnung: LAV (30 mm Durchmesser)
Probenhintergrund: Unterlagepapier ohne optischen Aufheller, Prüfklima: 21 °C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen in einer Waschmaschine) (60°C, 2h, Ariel Compact Pulverwaschmittel, saubere Beiladung).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät DATACOLOR 800 (Datacolor International).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

### Ergebnis

| | **Referenz** | **Mit Citronellol** |
|---|---|---|
| dB-Wert vor dem Waschen | 10,23 | 9,82 |
| dB-Wert nach dem Waschen | 4,86 | 4,24 |

**Fazit:** Die Baumwolllappen mit den Zubereitungen mit Citronellol zeigen nach dem Waschen eine geringere Gelbfärbung als die Baumwolllappen der Referenz.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.
1) Sonnenschutz-Lotion SPF 50

| **INCI** | **m (%)** |
|---|---|
| Tocopheryl Acetate | 0.06 |
| Glycyrrhetinic Acid | 0.1 |
| Polyglyceryl-2 Caprate | 0.3 |
| Hydrogenated Coco-Glycerides | 1 |
| Polyglyceryl-10 Stearate | 0.6 |
| Glyceryl Stearate | 1 |
| Silica Dimethyl Silylate | 0.5 |
| VP/Hexadecene Copolymer | 0.5 |
| Parfum (enthaltend Citronellol) | 0.6 ( 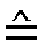 0.02% Citronellol) |
| Glycerin + Aqua | 0.9 |
| Aqua + Sodium Hydroxide | 0.2 |
| Phenoxyethanol | 0.3 |
| Behenyl Alcohol | 0.5 |
| Cellulose Gum | 0.2 |
| Hydroxyethylcellulose | 0.1 |
| Xanthan Gum | 0.4 |
| Sodium Polyacrylate | 0.08 |
| Hydroxypropyl Methylcellulose | 0.2 |
| Aqua | 52.86 |
| Alcohol Denat. + Aqua | 7 |
| Aqua + Trisodium EDTA | 1 |
| Tetrasodium Iminodisuccinate | 0.1 |
| Homosalate | 9.5 |
| Ethylhexyl Salicylate | 4.75 |
| Butyl Methoxydibenzoylmethane | 4.75 |
| Octocrylene | 8 |
| Titanium Dioxide (nano) + Silica + Dimethicone | 4 |
| Phenylbenzimidazole Sulfonic Acid | 0.5 |

2) Sonnenschutz-Spray SPF 50

| **INCI** | **m(%)** |
|---|---|
| Tocopheryl Acetate | 0.06 |
| Polyglyceryl-2 Caprate | 0.3 |
| Glycyrrhetinic Acid | 0.1 |
| Ethylhexylglycerin | 0.3 |
| Dibutyl Adipate | 52 |
| Copernicia Cerifera Cera | 1.5 |
| Polyglyceryl-10 Stearate | 0.5 |
| Triacontanyl PVP | 1 |
| VP/Hexadecene Copolymer | 1 |
| Parfüm (enthaltend Citronellol) | 1 ( 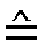 0.2% Citronellol) |
| Glycerin + Aqua | 1 |
| Aqua + Sodium Hydroxide | 0.1 |
| Phenoxyethanol | 0.5 |
| Cellulose Gum | 10 |
| Acrylates/C10-30 Alkyl Acrylate Crosspolymer | 0.1 |
| Xanthan Gum | 0.12 |
| Sodium Polyacrylate | 0.05 |
| Microcrystalline Cellulose + Cellulose Gum | 1 |
| Aqua | 58.12 |
| Alcohol Denat. + Aqua | 5 |
| Aqua + Trisodium EDTA | 1 |
| Tetrasodium Iminodisuccinate | 0.75 |
| Homosalate | 9 |
| Ethylhexyl Salicylate | 41755 |
| Bis-Ethylhexyloxyphenol Methoxyphenyl | |
| Triazine | 3 |
| Ethylhexyl Triazone | 3 |
| Butyl Methoxydibenzoylmethane | 4.75 |
| Phenylbenzimidazole Sulfonic Acid | 0.5 |

3) Sonnenschutz-Lotion SPF 10:

| **INCI** | **m(%)** |
|---|---|
| Tocopheryl Acetate | 0.06 |
| Glycyrrhetinic Acid | 0.1 |
| Polyglyceryl-2 Caprate | 0.3 |
| Ethylhexylglycerin | 0.15 |
| Hydrogenated Coco-Glycerides | 1 |
| Polyglyceryl-10 Stearate | 0.6 |
| Glyceryl Stearate | 1 |
| Silica Dimethyl Silylate | 1 |
| Tapioca Starch + Aqua | 1 |
| VP/Hexadecene Copolymer | 0.5 |
| Parfum (enthaltend Citronellol) | 0.5 ( 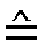 0.04% Citronellol) |
| Glycerin + Aqua | 0.9 |
| Phenoxyethanol | 0.3 |
| Myristyl Alcohol | 0.75 |
| Cellulose Gum | 0.5 |
| Hydroxyethylcellulose | 0.3 |
| Xanthan Gum | 0.4 |
| Sodium Polyacrylate | 0.1 |
| Aqua | 67.04 |
| Alcohol Denat. + Aqua | 7 |
| Aqua + Trisodium EDTA | 1 |
| Tetrasodium Iminodisuccinate | 0.5 |
| Homosalate | 4 |
| Ethylhexyl Salicylate | 3 |
| Butyl Methoxydibenzoylmethane | 1.5 |
| Octocrylene | 6.5 |

## Patentansprüche

1. Kosmetische Zubereitung enthaltend
a) ein oder mehrere UV-Filter,
b) Polyglyceryl-10 Stearat (INCI Polyglyceryl-10 Stearate),
c) Citronellol, wobei die Zubereitung frei ist von 2-Hydroxy-4-methoxybenzophenon, 4-Methoxyzimtsäure(2-ethylhexyl)ester, 4-Methoxyzimtsäureisoamylester und 3-(4-Methylbenzyliden)campher.

2. Verfahren zur Erhöhung der Auswaschbarkeit von organischen, öllöslichen UV-A Filtern und/oder organischen, öllöslichen Breitbandfiltern aus Textilien, die mit einer kosmetischen Zubereitung, welche diese UV-A Filter und/oder Breitbandfilter enthält, kontaminiert sind, **dadurch gekennzeichnet, dass** der kosmetischen Zubereitung Polyglyceryl-10 Stearat (INCI Polyglyceryl-10 Stearate) und Citronellol zugesetzt wird, wobei die Zubereitung frei ist von 2-Hydroxy-4-methoxybenzophenon, 4-Methoxyzimtsäure(2-ethylhexyl)ester, 4-Methoxyzimtsäureisoamylester und 3-(4-Methylbenzyliden)campher,

3. Verfahren zur Reduzierung der durch UV-Lichtschutzfilter enthaltenden kosmetischen Zubereitungen hervorgerufenen Textilverfleckung, **dadurch gekennzeichnet, dass** dem Kosmetikum Polyglyceryl-10 Stearat (INCI Polyglyceryl-10 Stearate) und Citronellol zugesetzt wird, wobei die Zubereitung frei ist von 2-Hydroxy-4-methoxybenzophenon, 4-Methoxyzimtsäure(2-ethylhexyl)ester, 4-Methoxyzimtsäureisoamylester und 3-(4-Methylbenzyliden)campher.

4. Verwendung einer Mischung aus Polyglyceryl-10 Stearat (INCI Polyglyceryl-10 Stearate) und Citronellol in UV-Lichtschutzfilter enthaltenden kosmetischen Zubereitungen zur Erleichterung der Auswaschbarkeit der UV-Lichtschutzfilter aus mit den Zubereitungen kontaminierten Textilien, wobei die Zubereitung frei ist von 2-Hydroxy-4-methoxybenzophenon, 4-Methoxyzimtsäure(2-ethylhexyl)ester, 4-Methoxyzimtsäureisoamylester und 3-(4-Methylbenzyliden)campher.

5. Kosmetische Zubereitung, Verfahren oder Verwendung nach einem der vorhergehenden Ansprüche, **dadurch gekennzeichnet, dass** die Zubereitung in Form einer ÖI-in-Wasser-Emulsion (O/W-Emulsion) vorliegt.

6. Kosmetische Zubereitung, Verfahren oder Verwendung nach einem der vorhergehenden Ansprüche, **dadurch gekennzeichnet, dass** die Zubereitung Polyglyceryl-10 Stearat in einer Konzentration von 0.1 bis 1.5 Gewichts-%, bezogen auf das Gesamtgewicht der Zubereitung enthält.

7. Kosmetische Zubereitung, Verfahren oder Verwendung nach einem der vorhergehenden Ansprüche, **dadurch gekennzeichnet, dass** die Zubereitung Citronellol in einer Konzentration von 10 ppm bis 0,3 Gewichts-%, bezogen auf das Gesamtgewicht der Zubereitung enthält.

8. Kosmetische Zubereitung, Verfahren oder Verwendung nach einem der vorhergehenden Ansprüche, **dadurch gekennzeichnet, dass** die Zubereitung Phenoxyethanol enthält.

9. Kosmetische Zubereitung, Verfahren oder Verwendung nach einem der vorhergehenden Ansprüche, **dadurch gekennzeichnet, dass** die Zubereitung Cellulosegummi (INCI Cellulose gum) und/oder Xanthangummi (INCI Xanthan gum) enthält.

10. Kosmetische Zubereitung, Verfahren oder Verwendung nach einem der vorhergehenden Ansprüche, **dadurch gekennzeichnet, dass** die Zubereitung Tetranatriumiminodisuccinat (INCI Tetrasodium Iminodisuccinate) enthält.

11. Zubereitung, Verfahren oder Verwendung nach einem der vorhergehenden Ansprüche, **dadurch gekennzeichnet, dass** die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

12. Zubereitung, Verfahren oder Verwendung nach einem der vorhergehenden Ansprüche, **dadurch gekennzeichnet, dass** die Zubereitung keine Parabene, sowie kein Methylisothiazolinon, Chlormethylisothiazolinon und DMDM-Hydantoin enthält, also frei ist von diesen Inhaltsstoffen.

13. Zubereitung, Verfahren oder Verwendung nach einem der vorhergehenden Ansprüche, **dadurch gekennzeichnet, dass** die Zubereitung einen oder mehrere UV-Filter gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan, 2-Phenylbenzimidazol-5-sulfonsäuresalzen, 2,4,6-Tris-[anilino-(p-carbo-2'-ethyl-1'-hexyloxy)]-1,3,6-triazin (INCI: Ethylhexyl Triazone), 2,4-Bis-{[4-(2-ethyl-hexyloxy)-2-hydroxy]-phenyl}-6-(4-methoxyphonyl)-1,3,5-triazin (INCI: Bis- Ethylhexyloxyphenol methoxyphenyl Triazine), 4,4'-[[6-[[4-[[(1,1 dimethylethyl)amino]carbonyl]phenyl]amino]-1,3,5-triazine-2,4-diyl]diimino]bis-, bis(2-ethylhexyl)benzoat (INCI: Diethylhexyl Butamido Triazone), 2-Ethylhexyl 2-hydroxybenzoat (INCI: Ethylhexyl Salicylate), 3,3,5-Trimethylcyclohexy) 2-hydroxybenzoat (INCI: Homosalate), Titandioxid enthält.

14. Zubereitung, Verfahren oder Verwendung nach einem der vorhergehenden Ansprüche, **dadurch gekennzeichnet, dass** die Zubereitung einen oder mehrere UV-Filter gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert-Butyl)-4'-methoxydibenzoylmethan, 2,4-Bis-{[4-(2-ethy)-hexyloxy)-2-hydroxy]-phenyl}-6-(4-mothoxyphonyl)-1,3,5-triazin (INCI: Bis- Ethyrhexyloxyphenol methoxyphenyl Triazine), enthält.

15. Zubereitung, Verfahren oder Verwendung nach einem der vorhergehenden Ansprüche, **dadurch gekennzeichnet, dass** die Zubereitung Titandioxid enthält.

## Claims

1. Cosmetic preparation comprising
a) one or more UV filters
b) polyglyceryl-10 stearate (INCI Polyglyceryl-10 Stearate)
c) citronellol, wherein the preparation is free from 2-hydroxy-4-methoxybenzophenone, 2-ethylhexyl 4-methoxycinnamate, isoamyl 4-methoxycinnamate and 3-(4-methylbenzylidene)camphor.

2. Method for enhancing the ability of organic, oil-soluble UVA filters and/or organic, oil-soluble broadband filters to be washed out of textiles which have been contaminated with a cosmetic preparation comprising these UVA filters and/or broadband filters, **characterized in that** polyglyceryl-10 stearate (INCI Polyglyceryl-10 Stearate) and citronellol are added to the cosmetic preparation, wherein the preparation is free from 2-hydroxy-4-methoxybenzophenone, 2-ethylhexyl 4-methoxycinnamate, isoamyl 4-methoxycinnamate and 3-(4-methylbenzylidene)camphor.

3. Method for reducing textile staining caused by cosmetic preparations comprising UV light protection filters, **characterized in that** polyglyceryl-10 stearate (INCI Polyglyceryl-10 Stearate) and citronellol are added to the cosmetic, wherein the preparation is free from 2-hydroxy-4-methoxybenzophenone, 2-ethylhexyl 4-methoxycinnamate, isoamyl 4-methoxycinnamate and 3-(4-methylbenzylidene)camphor.

4. Use of a mixture of polyglyceryl-10 stearate (INCI Polyglyceryl-10 Stearate) and citronellol in cosmetic preparations comprising UV light protection filters for facilitating the ability of the UV light protection filters to be washed out of textiles contaminated with the preparations, wherein the preparation is free from 2-hydroxy-4-methoxybenzophenone, 2-ethylhexyl 4-methoxycinnamate, isoamyl 4-methoxycinnamate and 3-(4-methylbenzylidene)camphor.

5. Cosmetic preparation, method or use according to any of the preceding claims, **characterized in that** the preparation is in the form of an oil-in-water emulsion (O/W emulsion).

6. Cosmetic preparation, method or use according to any of the preceding claims, **characterized in that** the preparation comprises polyglyceryl-10 stearate at a concentration of 0.1 to 1.5% by weight, based on the total weight of the preparation.

7. Cosmetic preparation, method or use according to any of the preceding claims, **characterized in that** the preparation comprises citronellol at a concentration of 10 ppm to 0.3% by weight, based on the total weight of the preparation.

8. Cosmetic preparation, method or use according to any of the preceding claims, **characterized in that** the preparation comprises phenoxyethanol.

9. Cosmetic preparation, method or use according to any of the preceding claims, **characterized in that** the preparation comprises cellulose gum (INCI Cellulose Gum) and/or xanthan gum (INCI Xanthan Gum).

10. Cosmetic preparation, method or use according to any of the preceding claims, **characterized in that** the preparation comprises tetrasodium iminodisuccinate (INCI Tetrasodium Iminodisuccinate).

11. Preparation, method or use according to any of the preceding claims, **characterized in that** the preparation comprises ethylhexylglycerin, propylene glycol, butylene glycol, 2-methylpropane-1,3-diol, pentane-1,2-diol, hexane-1,2-diol, octane-1,2-diol and/or decane-1,2-diol.

12. Preparation, method or use according to any of the preceding claims, **characterized in that** the preparation does not comprise any parabens, and also no methylisothiazolinone, chloromethylisothiazolinone and DMDM hydantoin, i.e. is free from these ingredients.

13. Preparation, method or use according to any of the preceding claims, **characterized in that** the preparation comprises one or more UV filters selected from the group of compounds comprising hexyl 2-[4-(diethylamino)-2-hydroxybenzoyl]benzoate (INCI: Diethylamino Hydroxybenzoyl Hexyl Benzoate), 4-(tert-butyl)-4'-methoxydibenzoylmethane, 2-phenylbenzimidazole-5-sulfonic acid salts, 2,4,6-tris[anilino-(p-carbo-2'-ethyl-1'-hexyloxy)]-1,3,5-triazine (INCI:
Ethylhexyl Triazone), 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine), 4,4'-[[6-[[4-[[(1,1-dimethylethyl)amino]carbonyl]phenyl]amino]-1,3,5-triazine-2,4-diyl]diimino]bis-, bis(2-ethylhexyl)benzoate (INCI: Diethylhexyl Butamido Triazone), 2-ethylhexyl 2-hydroxybenzoate (INCI: Ethylhexyl Salicylate), 3,3,5-trimethylcyclohexyl 2-hydroxybenzoate (INCI: Homosalate), titanium dioxide.

14. Preparation, method or use according to any of the preceding claims, **characterized in that** the preparation comprises one or more UV filters selected from the group of compounds comprising hexyl 2-[4-(diethylamino)-2-hydroxybenzoyl]benzoate (INCI: Diethylamino Hydroxybenzoyl Hexyl Benzoate), 4-(tert-butyl)-4'-methoxydibenzoylmethane, 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine).

15. Preparation, method or use according to any of the preceding claims, **characterized in that** the preparation comprises titanium dioxide.

## Revendications

1. Préparation cosmétique contenant
a) un ou plusieurs filtres UV,
b) du stéarate de polyglycéryle-10 (INCI Polyglyceryl-10 Stearate),
c) du citronellol, la préparation étant exempte de 2-hydroxy-4-méthoxybenzophénone, de l'ester 2-éthylhexylique de l'acide 4-méthoxycinnamique, de l'ester isoamylique de l'acide 4-méthoxycinnamique et de 3-(4-méthylbenzylidène)camphre.

2. Procédé d'augmentation de la rinçabilité de filtres UV-A organiques liposolubles et/ou de filtres à large bande organiques, liposolubles, en textiles, qui sont contaminés par une préparation cosmétique qui contient ces filtres UV-A et/ou filtres à large bande, **caractérisé en ce qu'**on ajoute à la préparation cosmétique du stéarate de polyglycéryle-10 (INCI Polyglyceryl-10 Stearate) et du citronellol, la préparation étant exempte de 2-hydroxy-4-méthoxybenzophénone, de l'ester 2-éthylhexylique de l'acide 4-méthoxycinnamique, de l'ester isoamylique de l'acide 4-méthoxycinnamique et de 3-(4-méthylbenzylidène)camphre.

3. Procédé de réduction des taches sur textiles, provoquées par des préparations cosmétiques contenant des filtres de protection contre la lumière UV, **caractérisé en ce qu'**on ajoute au produit cosmétique du stéarate de polyglycéryle-10 (INCI Polyglyceryl-10 Stearate) et du citronellol, la préparation étant exempte de 2-hydroxy-4-méthoxybenzophénone, de l'ester 2-éthylhexylique de l'acide 4-méthoxycinnamique, de l'ester isoamylique de l'acide 4-méthoxycinnamique et de 3-(4-méthylbenzylidène)camphre.

4. Utilisation d'un mélange de stéarate de polyglycéryle-10 (INCI Polyglyceryl-10 Stearate) et de citronellol dans des préparations cosmétiques contenant un filtre de protection contre la lumière UV, pour faciliter la rinçabilité du filtre de protection contre la lumière UV, en textiles, contaminés par les préparations, la préparation étant exempte de 2-hydroxy-4-méthoxybenzophénone, de l'ester 2-éthylhexylique de l'acide 4-méthoxycinnamique, de l'ester isoamylique de l'acide 4-méthoxycinnamique et de 3-(4-méthylbenzylidène)camphre.

5. Préparation cosmétique, procédé ou utilisation selon l'une des revendications précédentes, **caractérisée en ce que** la préparation se présente sous forme d'une émulsion huile-dans-l'eau (émulsion H/E).

6. Préparation cosmétique, procédé ou utilisation selon l'une des revendications précédentes, **caractérisée en ce que** la préparation contient le stéarate de polyglycéryle-10 à une concentration de 0,1 à 1,5 % en poids par rapport au poids total de la préparation.

7. Préparation cosmétique, procédé ou utilisation selon l'une des revendications précédentes, **caractérisée en ce que** la préparation contient le citronellol à une concentration de 10 ppm à 0,3 % en poids par rapport au poids total de la préparation.

8. Préparation cosmétique, procédé ou utilisation selon l'une des revendications précédentes, **caractérisée en ce que** la préparation contient du phénoxyéthanol.

9. Préparation cosmétique, procédé ou utilisation selon l'une des revendications précédentes, **caractérisée en ce que** la préparation contient de la gomme de cellulose (INCI Cellulose gum) et/ou de la gomme xanthane (INCI Xanthan gum).

10. Préparation cosmétique, procédé ou utilisation selon l'une des revendications précédentes, **caractérisée en ce que** la préparation contient de l'iminodisuccinate tétrasodique (INCI Tetrasodium iminodisuccinate).

11. Préparation, procédé ou utilisation selon l'une des revendications précédentes, **caractérisée en ce que** la préparation contient de l'éthylhexylglycérol, du propylèneglycol, du butylèneglycol, du 2-méthylpropane-1,3-diol, du 1,2-pentanediol, du 1,2-hexanediol, du 1,2-octanediol et/ou du 1,2-décanediol.

12. Préparation, procédé ou utilisation selon l'une des revendications précédentes, **caractérisée en ce que** la préparation ne contient pas de parabens, ni de méthylisothiazolinone, ni de chlorométhylisothiazolinone, ni de DMDM-hydantoïne, c'est-à-dire est exempte de ces constituants.

13. Préparation, procédé ou utilisation selon l'une des revendications précédentes, **caractérisé en ce que** la préparation contient un ou plusieurs filtres UV choisis dans le groupe des composés 2-[4-(diéthylamino)-2-hydroxybenzoyl]benzoate d'hexyle (INCI : Diethylamino hydroxybenzoyl hexyl benzoate), 4-(tert-butyl)-4'-méthoxydibenzoylméthane, sels de l'acide 2-phénylbenzimidazole-5-sulfonique, 2,4,6-tris-[anilino-(p-carbo-2'-éthyl-1'-hexyloxy)]-1,3,5-triazine (INCI : Ethylhexyl Triazone), 2,4-bis-{[4-(2-éthyl-hexyloxy)-2-hydroxy]-phényl}-6-(4-méthoxyphényl)-1,3,5-triazine (INCI : Bis-Ethylhexyloxyphenol methoxyphenyl Triazine), benzoate de 4,4'-[[6-[[4-[[(1,1-diméthyléthyl)amino]carbonyl]phényl]amino]-1,3,5-triazine-2,4-diyl]diimino]bis-,bis(2-éthylhexyle) (INCI : Diethylhexyl Butamido Triazone), 2-hydroxybenzoate de 2-éthylhexyle (INCI : Ethylhexyl Salicylate), 2-hydroxybenzoate de 3,3,5-triméthylcyclohexyle (INCI : Homosalate), dioxyde de titane.

14. Préparation, procédé ou utilisation selon l'une des revendications précédentes, **caractérisé en ce que** la préparation contient un ou plusieurs filtres UV choisis dans le groupe des composés 2-[4-(diéthylamino)-2-hydroxybenzoyl]benzoate d'hexyle (INCI : Diethylamino hydroxybenzoyl hexyl benzoate), 4-(tert-butyl)-4'-méthoxydibenzoylméthane, 2,4-bis-{[4-(2-éthyl-hexyloxy)-2-hydroxy]-phényl}-6-(4-méthoxyphényl)-1,3,5-triazine (INCI : Bis-Ethylhexyloxyphenol methoxyphenyl Triazine).

15. Préparation, procédé ou utilisation selon l'une des revendications précédentes, **caractérisé en ce que** la préparation contient du dioxyde de titane.
